# EUROPEAN PATENT APPLICATION

(11) **EP 4 437 995 A1**
(43) Date of publication of application: **02.10.2024**
(21) Application number: 23315071.3
(22) Date of filing: 31.03.2023
(51) Int. Cl.: A61B 34/20, A61B 34/00, A61B 17/00, A61B 34/30

(54) **A ROBOTIC INSTRUMENT MANIPULATION MODULE FOR MANIPULATION OF MEDICAL INSTRUMENTS**

(71) Applicant: Caranx Medical, 75013 Paris (FR)
(72) Inventor: BERTHET-RAYNE, Pierre, 06800 Cagnes-sur-Mer (FR); CERRUTI, Giulio, 06700 Saint-Laurent-Du-Var (FR); SCHEGG, Pierre, 06000 Nice (FR); SMITS, Jonas Victor Harmen, 3360 Bierbeek (BE)
(74) Representative: Hepp Wenger Ryffel AG

(57) **Abstract**

Robotic instrument manipulation module (101) for manipulating medical instruments (), comprising a manipulation unit (2) which has at least one translational driver (3) adapted for translationally moving the medical instrument (102) along a longitudinal axis (L) of the medical instrument and a rotational driver (5) for rotating the medical instrument (102) around its longitudinal axis. The manipulation module (101) has a centering unit (4) which is adapted for aligning the longitudinal axis of the medical instrument (102) in a two-dimensional plane orthogonal to the medical instrument (102) with a pre-defined axis (P), wherein the manipulation module (101) has a control unit (11) which is adapted for operating the manipulation unit (2), the rotational driver (5), and in particular the centering unit (4).

## Description

The invention relates to a robotic instrument manipulation module for manipulation of medical instruments according to the independent claims.

In the prior art, surgical intravascular procedures, particularly procedures involving crossing of the native heart valve such as transcatheter aortic valve implantation/replacement (TAVI/TAVR), are often performed by manually intraluminally guiding medical instruments such as catheters, dilators, or guidewires, e.g. through the femoral artery in a retrograde fashion to the aortic valve.

Medical instrument placement regarding translational advancement/retraction and rotation of the medical instrument must be particularly precise during such procedures, as these procedures carry significant health risks, such as detachment of intravascular plaque. These surgical interventions typically require the presence of a highly skilled clinician, who is thus not protected from imaging radiation, e.g. from an angiography device, associated with the procedure. Furthermore, successfully carrying out the surgical intervention is dependent on sensory fine motor skills of the clinician and may be subject to human error.

US 2021030492 A1 discloses a robotic catheter procedure system which has a drive mechanism to impart an axial force on a percutaneous device and has a rotational drive assembly for rotating a guide wire about its longitudinal axis.

WO 2018/189473 A1 discloses a drive module for an elongated flexible medical device which has a piezoelectric actuator formed by stacked elements including a drive surface and a base serving as a fixed reference and the drive surface is adapted for driving the elongated flexible medical device.

However, the prior art lacks a module with a simple design which allows for easier automated manipulation of medical instruments. Moreover, the prior art fails to provide a module which enables partially or fully automated alignment of medical instrument and a rotational and a translational movement of a medical instrument. In particular, a robotic instrument manipulation module should allow simplified and facilitated aligning and changing of different medical instruments and provide an active mechanism which is not reliant on the clinician's skill/attention or a learning curve for the manipulation of these medical instruments.

It is the object of the present invention to overcome these and other disadvantages of the prior art and provide an at least partially automated robotic instrument manipulation module for manipulating medical instruments defined in the independent claims. Further embodiments result from the dependent claims.

A robotic instrument manipulation module for manipulating medical instrument according to the invention comprises a manipulation unit which has at least one translational driver adapted for translationally moving the medical instrument along a longitudinal axis of the medical instrument. The manipulation module has a rotational driver for rotating the medical instrument around its longitudinal axis. The manipulation module has a centering unit which is adapted for aligning the longitudinal axis of the medical instrument in a two-dimensional plane orthogonal to the medical instrument with a pre-defined axis. The manipulation unit has a control unit which is adapted for operating the manipulation unit, the rotational driver, and in particular the centering unit.

The medical instrument may comprise or consist of an interventional or diagnostic instrument, in particular an imaging instrument, a drug delivery instrument, a sensory instrument, and/or a surgical instrument such as a catheter, a dilator, a sheath, a guidewire, a puncturing element, such as a cannula, and an implantation device.

The control unit may be adapted for operating the manipulation unit, the rotational driver, and in particular the centering unit in an at least partly automated manner, preferably in a fully automated manner, i.e. not being actively controlled by a clinician.

The alignment of the longitudinal axis of the medical instrument with a pre-defined axis allows for a more versatile functionality of the manipulation module and in particular facilitated and simplified changing of medical instruments.

The translational driver may have a first and second driving element for engaging the medical instrument, in particular formed by a first and a second cylindrical roller which are arrangeable on opposing sides of the medical instrument and are adapted for rotating in opposing directions, in particular around a symmetry axis of the cylindrical rollers, for translationally moving the medical instrument along the longitudinal axis.

This translational driver enables a longitudinal translational movement of the medical instrument with a high precision.

The alignment/movement of an element of this manipulation module, such as the contacting surface or the medical instrument, along a given axis or direction in the context of this application may be within a certain variation/tolerance, in particular within 1°, 5°, 10°, or 15° angular variation/tolerance with respect to the given axis or direction.

The first and second driving element may be convertible such that an interdistance between the first and second driving element may be changed to radially engage medical instrument with different cross-sectional dimensions. In addition, by adjusting the interdistance between the driving elements, the frictional contact with the medical instrument can be adjusted to minimize slippage and the applied force of the medical instrument, e.g. via the control unit. The control unit may be connected to a force sensor, in particular connected to the translational driver, which may measure the force exerted on the medical instrument.

Alternatively or additionally, the force sensor or a separate slippage sensor connected to the control unit may be adapted to determine if slippage of the medical instrument occurs during translation and/or rotation of the medical instrument. The sensor may further be adapted to determine the amount of slippage of the medical instrument such that the positioning of the medical instrument may be corrected accordingly by the control unit's operation.

The use of only a first and a second driving element may allow for simplified radial engagement of the medical instrument. However, the translational driver may comprise the first driving element, the second driving element and a third driving element for radially engaging the medical instrument at different circumferential positions at a specific location of the medical instrument.

Alternatively or additionally, the translational driver may comprise a first and second driving element which are adapted to engage the medical instrument at a first location and a third and fourth driving element adapted to engage the medical instrument at a second location different from the first location. This allows particularly reliable translational movement mechanisms of the medical instrument, e.g. an inchworm mechanism.

The driving elements may be individually actuated or functionally coupled by a translational coupling with each other such that only one actuator of the translational driver may operate the driving elements in unison and thus simplifying the complexity of the translational driver.

The rotational driver may be adapted for moving a first contacting surface and a second contacting surface of the translational driver in substantially parallel directions substantially orthogonally to the longitudinal axis of the medical instruments, in particular in opposing parallel directions, for rotating the medical instrument.

This rotational driver allows for a precise tuneable rotation of the medical instrument. The contacting surfaces may be arranged on the driving elements of the translational driver which reduces the complexity of the manipulation module by requiring less motorized components for engaging the medical instrument.

The contacting surfaces may be individually actuated or functionally coupled by a rotational coupling with each other such that only one actuator of the rotational driver may operate the contacting surfaces in unison and thus simplifying the complexity of the rotational driver.

The manipulation unit may be arranged on a rotatably mounted carrier. The rotational driver may be adapted for rotating the carrier and the medical instrument together with the manipulation unit, in particular together with the centering unit, in the two-dimensional plane orthogonal to the pre-defined axis.

This enables particularly precise motor alignment of the angular rotation of the medical instrument due to a large distance between the rotational driver and an engagement section of the medical instrument via the manipulation unit.

The rotational driver may be adapted such that a center of rotation of the rotational driver coincides with the pre-defined axis.

The rotational driver may have a circular shape coaxially arrangeable around the medical instrument and in particular the translational driver.

The rotational driver may include a gear system or a conveyor belt extending in particular at least partially along the circular shape of the rotational driver.

The translational driver may be formed by a rack pinion system, a screw nut system, a subbing injector, an inchworm mechanism, or a printer belt for engaging the medical instrument and moving the medical instrument along the longitudinal axis.

The translational driver may comprise at least one, in particular a plurality of piezo, a hydraulic, and/or pneumatic elements which are adapted for engaging the medical instrument.

These translational drivers allow for secure engagement of the medical instrument and in particular a distance of the translation movement can be particularly well controlled, adjusted, and monitored by the control unit.

The translational driver may be formed by a fluid injector and a flow tube which has a fluid inlet for receiving the fluid injector.

The translational driver may be formed by a rotatable spool or a capstan wheel for holding the medical instrument so that the medical instrument can be translationally moved along the longitudinal axis, in particular by the fluid injector.

The fluid injector can allow for rapid advancement and retraction of medical instrument when moving the medical instrument along its longitudinal axis. However, the monitoring and adjustability of a translational distance of the medical instrument' is not as controllable as previously described embodiments of the translational driver.

Combining the fluid injector with a capstan wheel or a rotating spool, whose bearings may be subject to some friction, may allow the adverse effects of the fluid injector to be minimized. In addition, the capstan wheel or the rotatable spool enables space-saving and secure positioning of the medical instrument.

The manipulation unit may have a longitudinal translation unit such that the translational driver, which is adapted for engaging the medical instrument, and in particular the centering unit, is movable together with the medical instrument in a direction parallel to the pre-defined axis.

This may allow to engage the medical instrument with the translational driver and subsequently moving the medical instrument along the longitudinal axis via the longitudinal translational unit.

The centering unit may have a centering structure such that the medical instrument can be translationally moved across the centering structure by the manipulation unit and aligned in the two-dimensional plane orthogonal to the medical instrument with the pre-defined axis extending through the centering structure.

This passive centering unit which has a centering structure allows for centering the medical instrument along the pre-defined axis in a simple manner by reducing the complexity of the manipulation module, in particular by minimizing the number of movable parts of the manipulation module.

The centering unit, in particular the centering structure may comprise a funnel, a passive roller guide or an O-ring seal which may be arrangeable at a plurality of circumferential position of the medical instrument, in particular circumferentially completely enclosing the medical instrument. The centering structure may form a guiding channel.

The centering structure may be arranged at a proximal end and/or a distal end of the centering unit. The proximal and distal end with respect to this application are defined from a proximally operating clinician's view.

The centering unit may be actuatable between a receiving position for receiving the medical instrument and a closed position for substantially aligning the medical instrument with the pre-defined axis.

This allows to advance the medical instrument across the centering unit in the receiving position in which the centering unit is preferably formed and shaped to allow for advancement of the medical instrument in a plurality of different spatial trajectories. When the medical instrument was advanced across the centering unit, the centering unit in the closed position can precisely engage and center the medical instrument along the pre-defined axis.

Prior to aligning the medical instrument along the pre-defined axis, the centering unit may arrange the medical instrument along a channel axis formed by the centering unit in the closed position. Subsequently, the channel axis may be aligned with the pre-defined axis, e.g. via the manipulation unit and/or rotational driver.

The at least one centering unit, in particular the centering structure(s), can be arranged distally and/or proximally from the manipulation unit, in particular from the translational driver.

The centering unit may comprise or consist of a notched gripper, an actuatable jewelry wire straightener, at least one radial gripper for radially gripping the medical instrument, or at least one actuatable diaphragm, in particular a plurality of diaphragms arranged in sequence.

The centering unit may have a centering surface, in particular a centering surface which has helical grooves, two conical centering surfaces forming a V-shape, or a combination of three rollers arrangeable at different circumferential positions of the medical instrument. The centering surface may be arranged on the translational driver or formed by the translational driver such that the longitudinal axis of the medical instrument is arranged through a specific location of the centering surface in the two-dimensional plane if the medical instrument is translationally moved along the longitudinal axis by the translational driver.

This allows the medical instrument to be aligned along the specific location of the centering surface which may be movable to the pre-defined axis or arranged on the pre-defined axis.Consequently, aligning the longitudinal axis of the medical instrument along the pre-defined axis may be simplified. In addition, the complexity of the manipulation module may be reduced and/or production costs for separate motorized components minimized by arranging the centering surface on the translational driver, in particular on the driving elements.

The centering surface may have two helical grooves which have opposite left-/right-handed winding directions. The centering surface may have an annular recess extending circumferentially around the driving element at the specific location such that the medical instrument is retained at the specific location irrespective of further translational movement along the longitudinal axis.

The centering surface may have a conical shape, in particular forming a V-shaped centering surface. The cylindrical rollers/centering unit may have an hourglass shape which may be rotationally symmetric around its longitudinal axis.

This allows the medical instrument to be moved to a predefined center position when the cylindrical rollers/centering unit engage the medical instrument.

A center position of the centering surface may be recessed and/or extend only in a circumferential direction of the cylindrical roller/centering unit to retain the first medical instrument in the center position during further rotation of the cylindrical roller in both directions.

The manipulation module may have a first lateral driver and a second lateral driver which are arranged at an angle greater 5° with respect to each other, in particular perpendicular to each other. The first and second lateral drivers are adapted for substantially aligning the longitudinal axis of the medical instrument in the two-dimensional plane orthogonal to the medical instrument with the pre-defined axis.

The manipulation module may have the rotational driver which is adapted for rotating the medical instrument together with the manipulation unit. The manipulation module may have one lateral driver which is arranged to move the medical instrument, in particular with the manipulation unit,'in a direction orthogonally to the pre-defined axis, preferably running through a geometric center of the rotational driver. The rotational driver and the lateral driver are adapted for aligning the longitudinal axis of the medical instrument in the two-dimensional plane with the pre-defined axis.

The manipulation module comprising the two lateral drivers may allow for aligning the medical instrument in the two-dimensional plane along two translational degrees of freedom with the pre-defined axis.

The manipulation module comprising the rotational driver and one lateral driver allow for aligning the medical instrument in the two-dimensional plane with the pre-defined axis by a rotational degree of freedom and one translational degree of freedom.

The rotational driver may therefore perform two functions, namely rotating the medical instrument about its longitudinal axis and also acting as a component of the centering unit to align the longitudinal axis of the medical instrument along the pre-defined axis. This may reduce the complexity of the manipulation module.

The manipulation unit may be convertible between an engagement configuration for engaging the medical instrument and a release configuration for releasing the medical instrument. The control unit and the manipulation unit may be adapted for changing the medical instrument by releasing a first medical instrument by converting the manipulation unit from the engagement configuration to the release configuration. The control unit and the manipulation unit may be adapted for aligning the manipulation unit with a second medical instrument, in particular by a relative movement of the manipulation unit with respect to the second medical instrument in the two-dimensional plane orthogonal to the medical instruments. The control unit and the manipulation unit may be adapted for engaging the second medical instrument by converting the manipulation unit from the release configuration to the engagement configuration.

This increases the versatility of the manipulation module since the presence of a clinician for changing the medical instruments is not required.

The control unit may further be adapted to control the rotational driver for aligning the manipulation unit with a second medical instrument.

The control unit and the manipulation unit may be adapted for engaging and releasing the medical instruments in the engaging and release configuration in a radial direction respectively. This facilitates changing the medical instruments, due to e.g. avoiding the necessity for separate longitudinal insertion of medical instruments into the translational driver.

The manipulation module may have at least one position sensor for detecting a position of the medical instrument within the two-dimensional plane orthogonal to the medical instrument with respect to the manipulation module, in particular with respect to a position within the two-dimensional plane of the translational driver.

The position sensor may be adapted providing a feedback signal to the control unit. Based on the feedback signal indicative of the two-dimensional position of the medical instrument, the control unit may be operated to align the longitudinal axis of the medical instrument with the pre-defined axis.

The position sensor may comprise or consist of an optical sensor or a proximity sensor such as a capacitive sensor or an inductive sensor.

Alternatively or additionally, the position sensor may be adapted for detecting a distance of a longitudinal translation movement of the medical-instrument by the translational driver. The position sensor may be adapted for detecting an angular rotation of the medical instrument being rotated around its longitudinal axis by the rotational driver.

The manipulation module may have at least one instrument identification sensor for detecting a cross-sectional dimension of the medical instrument engaged by the translational driver in the engagement configuration. The control unit may be adapted to determine a classification of the medical instrument or a longitudinal subsection of a medical instrument with different cross-sectional dimensions at different longitudinal subsections via the instrument identification sensor.

This allows that the selection of medical instruments may be verified for the corresponding surgical procedural steps in an at least partially automated manner. In addition, the detection of changes in the cross-section of the medical instrument may be associated with, for example, a longitudinal position of the medical instrument.

The instrument identification sensor may be included in the translational driver.

The manipulation module may comprise a straightening means for straightening the medical instrument, in particular by the control unit being adapted to control an advancement of the medical instrument via the manipulation unit through the centering unit and subsequently retracting the medical instrument through the centering unit.

The straightening means may allow for simplified nesting of medical instruments within each other and/or straightening curved/pre-curved medical instruments such as catheters or guidewires, e.g. a pre-shaped "pigtail" TAVI/TAVR catheter or a j-tip wire or a safari (R) pacing wire prior to insertion. A pigtail catheter or a guidewire, in particular a SAFARI TAVI/TAVR wire, may have a distal end which is wound up into a curl to prevent slippage after positioning the catheter or wire.

This may facilitate and enable loading medical instruments within each other for surgical procedures requiring nested medical instruments.

The manipulation module may have a second manipulation unit which has at least one additional translational driver adapted for translationally moving a second medical instrument along a longitudinal axis of the second medical instrument. The manipulation module may have a second rotational driver for rotating the second medical instrument around its longitudinal axis. The manipulation module may have a second centering unit which is adapted for aligning the second longitudinal axis of the second medical instrument in a two-dimensional plane orthogonal to the second medical instrument with the pre-defined axis. The manipulation module may have a control unit which is adapted for operating the first and the second manipulation unit, the first and preferably the second rotational driver and in particular the first and second centering unit.

This allows for independent manipulation, i.e. independent alignment, translational and rotational movement, of the first and second medical instrument, in particular simultaneously. This may be required when carrying out sophisticated surgical interventions such as TAVI/TAVR, in a partially or fully automated manner.

The manipulation module may be adapted for allowing a backloading of the first medical instrument, e.g. a guidewire/catheter, into or over the second medical instrument, e.g. a catheter/catheter.

Alternatively, the manipulation module may only comprise one centering unit which is adapted for aligning the longitudinal axis of the first and second medical instrument in the two-dimensional plane orthogonal to the medical instruments with the pre-defined axis.

The invention is now described with reference to certain embodiments and figures which show:
- Figures 1A and 1B:: A perspective side view of an embodiment of the robotic instrument manipulation module having a translational driver attached to a carrier rotatably mounted on a rotational driver,
- Figure 2A:: A perspective side view of an embodiment of a centering element of the centering unit of Figs. 1A and 1B with an open shape,
- Figures 2B - 2C:: A top view of an embodiment of the centering unit formed by a plurality of stacked diaphragms in a closed position and receiving position,
- Figure 2D:: A perspective side view of an embodiment of the centering unit formed by an industrial wire straightener in a receiving position,
- Figure 2E:: A schematic representation of a first and a second lateral driver which are adapted for aligning the longitudinal axis of the medical instrument in a two-dimensional plane orthogonal to the medical instrument with the pre-defined axis,
- Figure 3A:: A perspective side view of a first embodiment of the manipulation unit having a translational driver and a centering unit spaces apart from each other which are adapted for translationally moving and aligning a medical instrument,
- Figure 3B:: A perspective side view of a second embodiment of the manipulation unit having a translational driver with cylindrical rollers on which a centering surface of a centering unit is arranged which is adapted for translationally moving and aligning a medical instrument,
- Figures 4A and 4B:: An embodiment of a driving element of the translational driver on which a centering surface of a centering unit is arranged with a medical instrument arranged at a helical section and the medical instrument arranged at the specific location of the centering surface,
- Figure 4C:: A perspective side view of a third embodiment of the manipulation unit having a translational driver with two longitudinally spaced apart driving elements of Figs. 4A and 4B adapted for translationally moving and aligning a medical instrument,
- Figures 4D-4H:: Six different perspective views of an embodiment of half a translational driver having a cylindrical roller with two conical centering surfaces forming a V-shape,
- Figure 4I:: A cross-sectional view of the cylindrical roller of Figs. 4D-4H,
- Figures 5A - 5F:: A cross-sectional view of six different embodiments of the translational driver of a manipulation module,
- Figure 5G:: A perspective side view of an embodiment of the translational driver of a manipulation module formed by a capstan wheel which has a proximally and distally arranged-centering unit,
- Figures 6A - 6H:: A cross-sectional view of an embodiment of the translational driver of a manipulation module formed by an inchworm mechanism for translationally moving a medical instrument,
- Figures 7A - 7B:: A cross-sectional view of an embodiment of the translational driver and rotational driver of a manipulation module formed by two cylindrical rollers rotating the medical instrument and a longitudinal-sectional view of the cylindrical rollers moving the medical instrument along its longitudinal axis,
- Figures 8A - 8C:: A cross-sectional view and a longitudinal-sectional view of an embodiment of the translational driver and rotational driver of the manipulation module formed by contacting surfaces for engaging a medical instrument connected to a solid support,
- Figure 9A:: A schematic representation of a manipulation unit of the manipulation module convertible between an engagement configuration and a release configuration,
- Figures 9B and 9C:: A first and second schematic representation of a manipulation unit of the manipulation module formed by a translational driver, a rotational driver and a longitudinal translation unit.

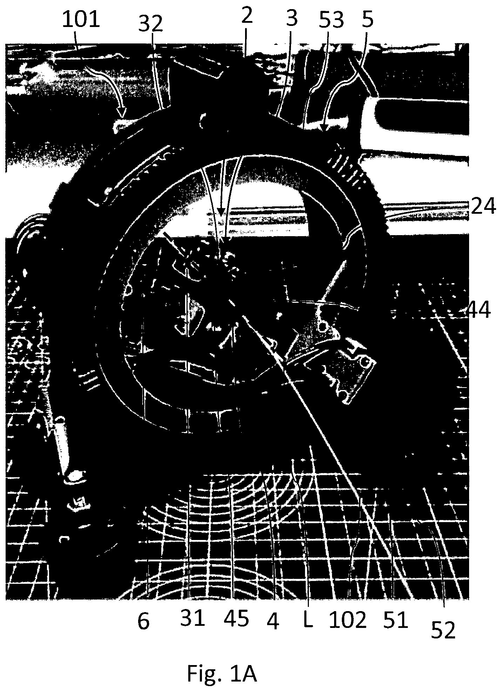

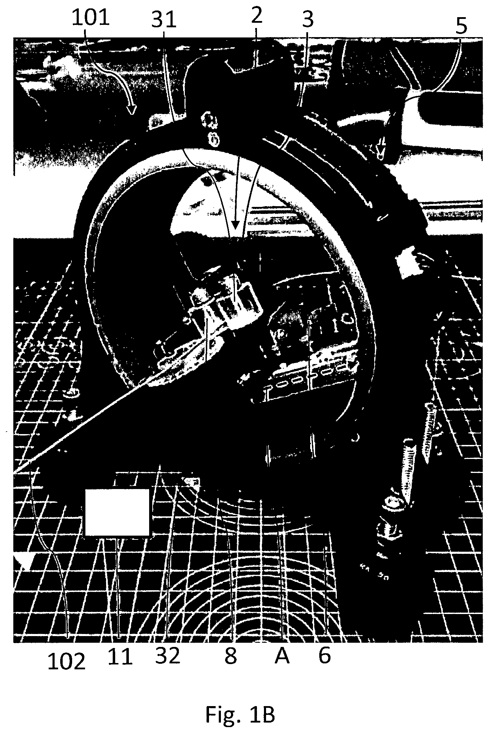

The figures 1A and 1B show a perspective side view of an embodiment of the robotic instrument manipulation module 101 having a manipulation unit 2 which has a translational driver 3 mounted on a carrier 6 which is rotatably mounted on a rotational driver 5. The figures 1A and 1B show a medical instrument 102 formed by a guidewire having a longitudinal axis L which is engaged by the translational driver 3 and arranged through a centering unit 4. The translational driver 3 and the centering unit 4 are arranged on the carrier 6 and are translationally movable along the carrier 6. The carrier 6 is arranged orthogonally to a pre-defined axis along which a longitudinal axis L of the medical instrument 102 shall be aligned by the robotic instrument manipulation module.

The translational driver and the centering unit in Figs. 1A and 1B are convertible by the control unit 11. The translational driver 3 is convertible between an engagement configuration 24 and a release configuration (not shown in Figs. 1A and 1B) by a lateral driver 8 (see Fig. 1B). The figures 1A and 1B show the translational driver 3 in the engagement configuration 24. The translational driver 3 has a first cylindrical roller 31 and a second cylindrical roller 32 which are movable independently from each other in a direction laterally to the longitudinal axis L of the medical instrument 102 to convert the translational driver 3 between the engagement configuration 24 and a release configuration.

The translational driver 3 comprises a rack pinion system 33 for driving a rotational movement of the first and second cylindrical roller 31, 32 in opposing directions for advancing or retracting the medical instrument 102 along the longitudinal axis L.

The centering unit 4 has a first centering element 44 and a second centering element 45 which may be coupled to the cylindrical rollers 31, 32. The centering elements 44, 45 in Figs. 1A and 1B are movable in unison together with the first and second cylindrical rollers 31, 32 of the translational driver 3 along the carrier 6 respectively.

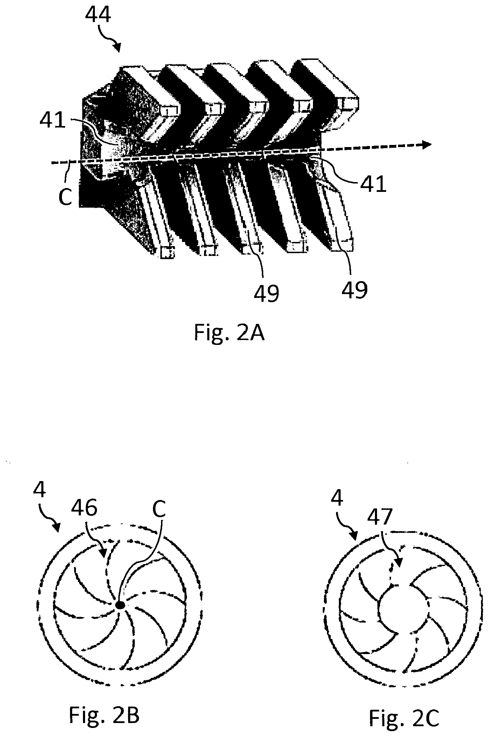

The first centering element 44 and the second centering element 45 have a plurality of teeth 49 respectively (see Fig. 2A) which each form an open triangular shape 47 facing toward each other. The centering unit 4 in Figs. 1A and 1B is convertible between the receiving position for receiving the medical instrument 102 and the closed position 46 for aligning the medical instrument 102 by moving the centering elements 44, 45 towards each other or apart from each other along the carrier 6. In Figs. 1A and 1B, the centering unit 4 is in the closed position 46.

In the receiving position (not shown in Figs. 1A and 1B), the centering elements 44, 45 are laterally spaced apart from the longitudinal axis of the medical instrument 102. In the closed position 46 in Figs. 1A and 1B, the teeth 49 of the centering unit 4 intermittently engage each other such that they partially overlap longitudinally to align the medical instrument 102 along a channel axis C formed by the centering unit 4 (see Fig. 2A). The channel axis C of the centering unit 4 along which the longitudinal axis of the medical instrument 102 in Figs. 1A and 1B was aligned is alignable along the pre-defined axis by moving the centering unit 4 together with the translational driver 3.

The rotational driver 5 in Figs. 1A and 1B has a circular shape and is driven by a second rack pinion system 51 which extends annularly around the medical instrument 102, the translational driver 3, and the centering unit 4. The rotational driver 5 is adapted for rotating the medical instrument 102 by rotating the carrier 6 on which the lateral driver 8, the translational driver 3, and the centering unit 4 are arranged. This movement can be achieved by a gear 52 of the second rack pinion system 51 moving circularly along a ring gear 53 of the rack pinion systems (see Fig. 1A).

The rotary movement via the rotational driver 5 and the lateral driver 8 for moving the translational driver 3 and the centering unit 4 along a lateral axis A (see Fig. 1B) allow for positioning the longitudinal axis L of the medical instrument 102 in a two-dimensional plane such that the medical instrument 102 can be aligned along the pre-defined axis. The two-dimensional plane is orthogonally arranged with respect to the pre-defined axis.

The manipulation unit 2, the rotational driver 5, the lateral driver 8, and the centering unit 4 are connected and operated by a control unit 11 of the manipulation module 101.

Figure 2A shows a perspective side view of an embodiment of the centering element 44 of the centering unit 4 of Figs. 1A and 1B with an open shape. A plurality of teeth 49 are arranged spaced apart along a channel axis C of the centering unit 4. The plurality of teeth 49 form a wedge open shape for laterally engaging the medical instrument (see Figs. 1A and 1B). The second centering element 45 in Figs. 1A and 1B is formed correspondingly to the first centering element 44 in Fig. 2A, except that it is oriented laterally reversed so that the teeth 49 can engage with each other.

The centering element 44 has a centering structure 41 on either side of the channel which is formed by a funnel shape such that the medical instrument can be easily guided into the channel of the centering unit 4. In the receiving position the centering elements 44, 45 may be laterally spaced apart from the medical instrument to allow for simple advancement of the medical instrument across the centering unit 4 in a plurality of trajectories or shapes of the medical instrument. In the closed position 46, the centering elements 44, 45 are adapted to engage the medical instrument radially.

This allows the teeth 49 of the first and second centering element 44, 45 may intermittently engage each other such that they are partially overlapping along the channel axis C and a longitudinal axis of the medical instrument is aligned with the channel axis C.

The figures 2B and 2C show a top view of an embodiment of the centering unit 4 formed by a plurality of stacked diaphragms in closed position 46 and a receiving position 47.

The diaphragms shown in figure 2B show a closed position 46 of the centering unit 4 and the diaphragms in figure 2C show a receiving position 47 which are convertible between each other by a control unit 11 (see Fig. 1B). A confined center of the diaphragms in the closed position 46 defines the channel axis C. The channel axis C in Fig. 2B is arranged or can be arranged with a pre-defined axis for centering the medical instrument.

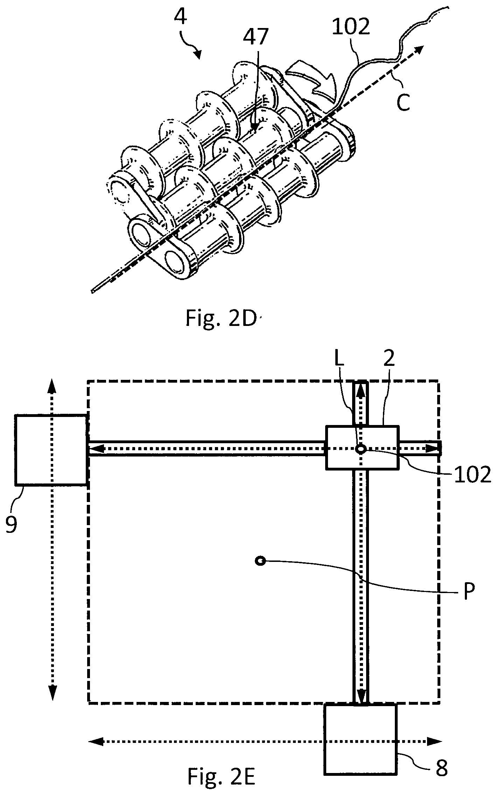

The figure 2D shows a perspective side view of an embodiment of the centering unit 4 formed by an industrial wire straightener in a receiving position 47. The centering unit 4 has three parallel struts with a plurality of annular protrusions. The parallel struts are connected to each other via hinges which may be arranged at the distal tips of the parallel struts.

The centering unit in figure 2D is connected to a rotational actuator (not shown in Fig. 2D) which is operable by the control unit (see Fig. 1B) to rotate the struts with respect to each other. This allows that the centering unit 4 is convertible from the receiving position 47 to a closed position for centering the medical instrument 102.

The radial protrusions are spaced apart from each other along a channel axis C such that each annular protrusion engages a medical instrument 102 in the closed position at different location of the channel axis C to align the medical instrument 102 along the channel axis C.

The control unit may be adapted to advance the medical instrument 102 across the centering unit 4 in Figs. 1A - 2D in the receiving position 47, convert the centering unit 4 to the closed position, and subsequently retract the medical instrument 102. This allows the medical instrument 102 to be straightened and aligned along the channel axis C.

The figure 2E shows a schematic representation of a first and a second lateral driver 8, 9 which are adapted for aligning the longitudinal axis L of the medical instrument 102 in a two-dimensional plane (shown as a dashed rectangular shape in Fig. 2E) orthogonal to the medical instrument 102 with the pre-defined axis P.The figure 2E shows that the lateral drivers 8, 9 are adapted for moving the manipulation unit 2 and centering unit together with the medical instrument 102 in the two-dimensional plane. The lateral drivers 8, 9 are connected to a control unit which is adapted for aligning the medical instrument in a partially or fully automated manner.

The control unit in Figs. 2A - 2D may be adapted to align the channel axis C of the centering unit 4 along the pre-defined axis as previously described by a rotational driver and a translational driver (see Figs. 1A and 1B) or two translational drives 8, 9 (see Fig. 2E).

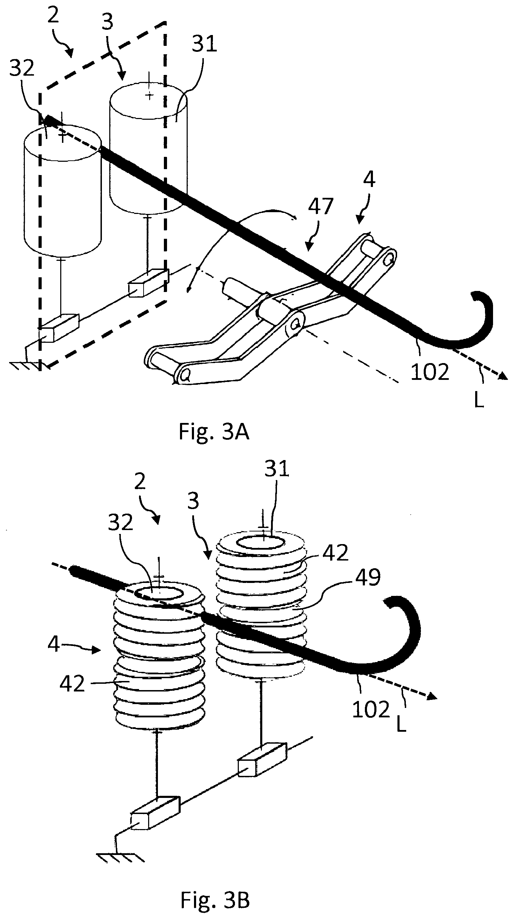

The figure 3A shows a perspective side view of a first embodiment of the manipulation unit 2 having a translational driver 3 and a centering unit 4 which are arranged spaced apart from each other along a longitudinal axis L of a medical instrument 102. The translational driver 3 is adapted for translationally moving the medical instrument 102 and the centering unit 4 is adapted for aligning the medical instrument 102.

The translational driver 3 is formed by a first and a second cylindrical roller 31, 32 as previously described in Figs. 1A and 1B. The cylindrical rollers 31, 32 can be operated via a control unit (not shown in Fig. 3A, see Fig. 1B) for advancing the medical instrument 102 across the centering unit 4. The centering unit 4 can be controlled via the control unit such that the centering unit 4 is in the receiving position 47 when the medical instrument 102 is advanced. This allows for easier advancement of the medical instrument 102 along a plurality of spatial trajectories. Subsequently, the centering unit 4 may be converted to a closed position, such that a longitudinal axis L of the medical instrument 102 may aligned with a pre-defined axis. The control unit may further be adapted to retract the medical instrument 102 through the centering unit 4 in the closed position for straightening the medical instrument 102.

The figure 3B shows a perspective side view of a second embodiment of the manipulation unit 2 having a translational driver 3 with a first and a second cylindrical roller 31, 32 on which a centering surface 42 of a centering unit 4 is arranged. The translational driver 3 is adapted for translationally moving a medical instrument 102. At the same time the centering surface 42 is adapted for centering a longitudinal axis L of the medical instrument 102 along a specific spatial location 49 of the centering surface 42. For this reason, each centering surface 42 of the cylindrical rollers 31, 32 has a left- and a right-handed helically recessed section. The helical recessed sections are adapted for transferring the medical instrument 102 into the specific spatial location 49 by advancing the medical instrument 102 along its longitudinal axis L. The specific spatial location 49 of the centering surface 42 forms a recessed section which only extends only in a circumferential direction along cylindrical rollers 31, 32. This allows that the medical instrument 102 remains within the specific spatial location 49 once being arranged in the specific spatial location 49 irrespective of further actuation via the translational driver 3. The centering surface 49 and the cylindrical rollers 31, 32 may be integrally formed with each other. The manipulation unit 2 and centering unit 4 in Figs. 3A and 3B may be rotatably mounted to a rotational driver of a manipulation module 101 (see Figs. 1A and 1B) .

A control unit (not shown in Figs. 3A and 3B) may be adapted to operate the manipulation unit 2 to adapt an interdistance between the cylindrical rollers 31, 32 of the translational driver 3 such that medical instruments 102 with different cross-sectional dimensions can be engaged and moved by the cylindrical rollers 31, 32.

In addition, a manipulation module comprising a manipulation unit 2 according to Figs. 3A or 3B may have a control unit which is adapted to either move a different medical instrument into the translational driver 3 or move the translational driver 3 to a different medical instrument.

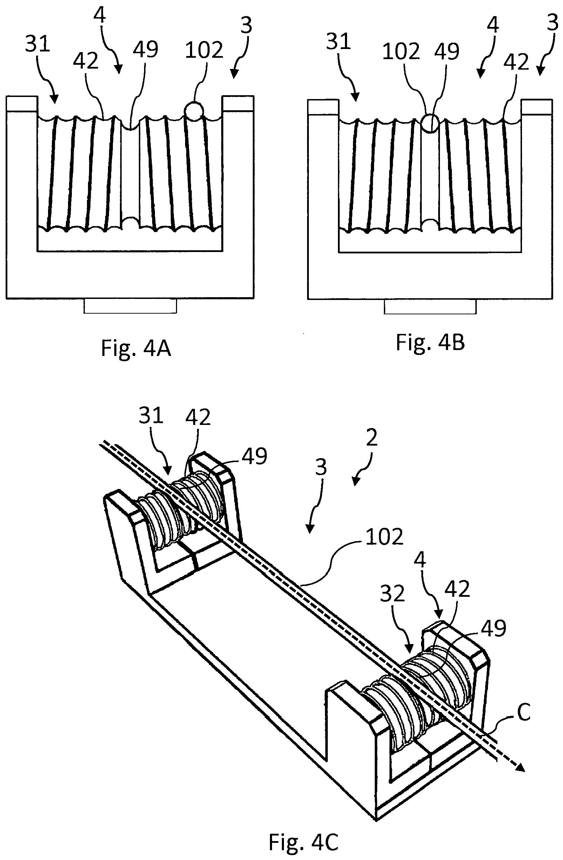

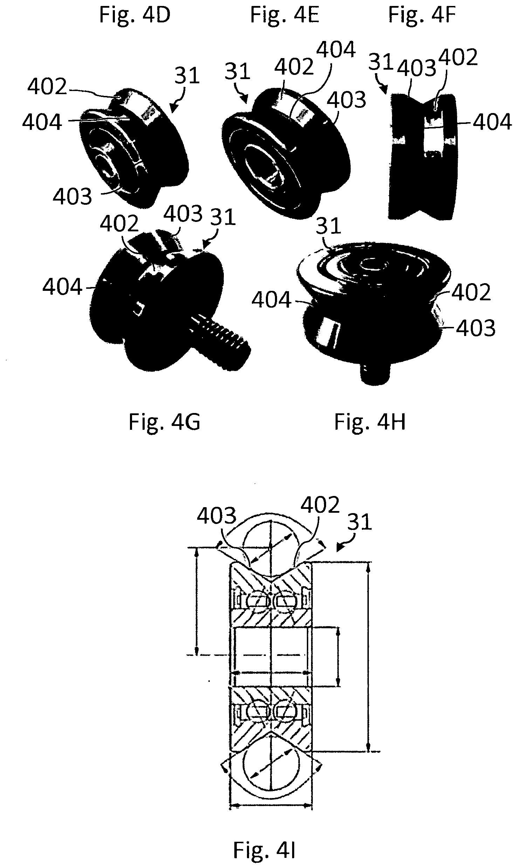

The figures 4A and 4B show a front view of an embodiment of a driving element 31 of the translational driver 3 on which a centering surface 42 of a centering unit 4 is arranged with a medical instrument 102 located at a helical section and the medical instrument 102 arranged at a specific location 49 of the centering surface 42. The driving element 31 is formed by a cylindrical roller as described in Fig. 3B.

The medical instrument 102 in Fig. 4A may be transferred by rotation of the driving element 31 in one direction, preferably a distal direction, from a helical section of the centering surface 42 of the centering unit 4 located on the driving element 31 to the specific location 49 shown in Fig. 4B. The specific location 49 in Figs. 4A and 4B extends only circumferential direction of the driving element 31 and is more recessed than the helical section. This can ensure that the medical instrument 102 remains in the specific location 49 during further rotation of the driving element 31, irrespective of a direction of rotation.

The figure 4C shows a perspective side view of a third embodiment of the manipulation unit 2 having a translational driver 3 with two longitudinally spaced apart driving elements 31, 32 formed as cylindrical rollers shown in Figs. 4A and 4B adapted for translationally moving and aligning a medical instrument 102. The driving elements 31, 32 formed by the cylindrical rollers and centering surfaces 42 of the centering unit 4 have the same shape and orientation of helical sections. This allows that a forward movement of the medical instrument 102 by rotating the driving elements 31, 32 in the same direction aligns the medical instrument 102 in the specific location 49 of the centering surface 42. The specific location 49 of the centering surface 42 are arranged along a channel axis C. A longitudinal axis of the medical instrument 102 can thus be aligned via the centering surfaces 42 with the channel axis C.

The channel axis C may be aligned along the pre-defined axis of a manipulation module. Alternatively, the manipulation unit 2 in Fig. 4C may be movable, e.g. by two translational drivers 8, 9 (see Fig. 2E) or a translational and a rotational driver (see Figs. 1A and 1B), such that the longitudinal axis / channel axis C of the medical instrument 102 is aligned in a two-dimensional plane orthogonal to the medical instrument 102 with a pre-defined axis.

A person skilled in the art would realize that the manipulation unit 2 in Fig. 4C may further comprise additional driving elements, which are preferably arranged on the laterally opposite side of the spaced apart driving elements 31, 32 in Fig. 4C. This allows that the medical instrument 102 may be engaged from either side as shown in Figs. 1A, 1B, and 3A.

In one embodiment, the spaced apart driving elements 31, 32 or pairs of driving elements 31, 32 may be adapted to be rotated independently from each other, in particular with a different speed / direction for straightening the medical instrument 102, preferably controlled by a control unit (see Fig. 1B). This may allow for example a distal pair of driving elements 32 to rotate faster than the proximal pair of driving elements 31 exerting a tension on the medical instrument 102 which may allow for straightening of the medical instrument 102.

The figures 4D-4H show six different perspective views of an embodiment of half a translational driver 3 having a driving element formed by a cylindrical roller 31 with two conical centering surfaces 402, 403 forming a V-shape. The V-shape of the two conical centering surfaces 402, 403 of the cylindrical roller 31 allows to center a medical instrument within a recessed center position 404 when engaging the medical instrument from opposing sides with two cylindrical rollers 31, 32 (see e.g. Fig. 3A). The figure 4I shows a cross-sectional view of the cylindrical roller 33 of Figs. 4D - 4H without a recessed center position.

The Figs. 4D - 4H show that the cylindrical roller 31 may have a centric passage or for receiving a spindle to be coupled to a rotational actuator. In addition, the cylindrical roller 31 may comprise a ball bearing.

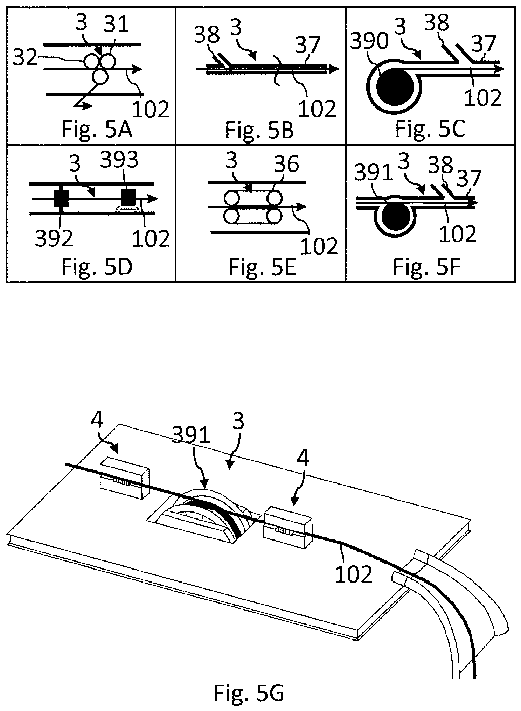

The figures 5A - 5F show a cross-sectional view of six different embodiments of the translational driver 3 of the manipulation module.

The figure 5A shows three driving elements 31, 32 formed by rollers which are arranged spaced apart from each other along a longitudinal axis of a medical instrument 102 indicated by the black arrow. The rollers are adapted for translationally moving the medical instrument 102 by rotating and may be adapted for centering and aligning the medical instrument 102 as previously described.

The figure 5B shows that the translational driver 3 is formed by a flow tube 37 which has a fluid inlet 38 for receiving a fluid injector. The translational driver 3 may also comprise the fluid injector. By injecting fluid with the fluid injector via the fluid inlet 38 into the flow tube 37, the medical instrument 102 may be translationally moved along its longitudinal axis in the flow tube 37.

The figure 5C shows that the translational driver 3 is formed by a flow tube 37 and a fluid inlet 38 as described in Fig. 5B. In addition, the medical instrument 102 is arranged wound up on a rotatable spool 390 which is enclosed by a housing which has an opening which is connected to the flow tube 37 in a fluid-connected manner. The spool 390 may be configured, e.g. due to a frictional resistance, to allow only a certain unwinding speed to allow the medical instrument 102 to move more controlled manner along the longitudinal axis.

The figure 5D shows that the translational driver 3 is formed by a snubbing injector 34 which has a stationary slip 392 and a travelling slip 393 which is adapted to be advanced and retracted along the longitudinal axis of the medical instrument 102. The stationary slip 392 and the travelling slip can be coupled to the medical instrument 102 by radially engaging the medical instrument 102. The stationary slip 392 and the travelling slip 393 are adapted for engaging the medical instrument 102 in sequence for actuating the medical instrument 102 in its longitudinal direction.

The figure 5E shows that the translational driver 3 is formed by a tractor injector formed by two printer belts 36 on two rollers respectively. The printer belts 36 are adapted to radially engage the medical instrument from opposing sides for actuating the medical instrument 102 along its longitudinal axis.

The figure 5F shows that the translational driver 3 of a manipulation module is formed by a flow tube 37 which has a fluid inlet 38 and a capstan wheel 391. The medical instrument 102 is wound up on the capstan wheel 391 and extends proximally and distally from the capstan wheel 391 into the flow tube 37. A housing arranged around the capstan wheel has a proximal entry opening and a distal exit opening connected to the flow tube 37. The medical instrument 102 can be fed to the capstan wheel 391 from the proximal entry opening and led away from the capstan wheel 391 from the distal exit opening. The fluid inlet 38 is arranged distally from the distal exit opening of the housing. The capstan wheel 391 may be configured to allow only a certain unwinding speed to allow the medical instrument 102 to move more smoothly along the translational degree of freedom. The capstan wheel 391 may be formed to provide a specific force resistance, such that movement of the medical instrument 102 is only possible through greater force transmission of a fluid injector connected to the fluid inlet 37. In one embodiment, the capstan wheel 391 may comprise an adjustable transmission which may allow for adapting the force transmission for moving the medical instrument, such that the capstan wheel 391 may be adapted for performing a longitudinal translation of the medical instrument 102 at different speeds / precisions.

The figure 5G shows an embodiment of the translational driver 3 formed by a capstan wheel 391 which has a proximally and distally arranged centering unit 4. The capstan wheel 391 in Fig. 5G is motorized to be rotated for moving a medical instrument 102 in its longitudinal direction L.

The translational drivers 3 in Figs. 5A - 5G may be connected to and controlled by a control unit. The translational drivers 3 in Figs. 5A - 5G, and in particular the centering unit(s) 4, may be mounted on a rotational driver to allow for the rotation of the medical instrument around its longitudinal axis.

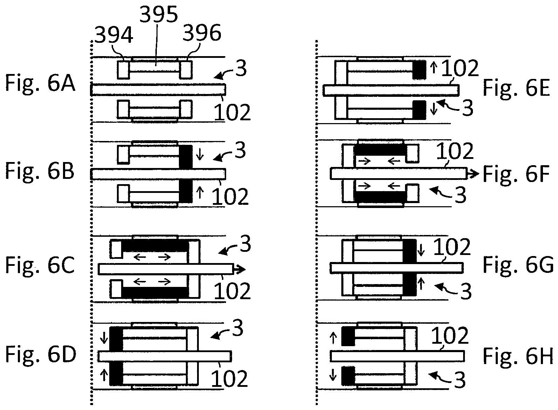

The figures 6A - 6H show a cross-sectional view of a translational driver 3 of a manipulation module which is formed by an inchworm mechanism for translationally moving a medical instrument 102 along its longitudinal axis. The inchworm mechanism in Figs. 6A - 6H has a proximal, an intermediate, and a distal piezoelectric element 394, 395, 396. The piezoelectric elements on the inchworm mechanisms on the opposite side of the medical instrument 102 are formed analogously and controlled simultaneously and in the same manner.

The distal piezoelectric element 396 is adapted to be deformed in a radial direction with respect to the medical instrument 102 such that it may frictionally engage and release the medical instrument 102 (see Figs. 6B, 6E, and 6G).

The intermediate piezoelectric element 395 is adapted to be deformed parallel to the longitudinal axis of the medical instrument 102 such that a distance between the proximal and distal piezoelectric element may be changed (see Figs. 6C and 5F). This may analogously be achieved by a translational actuator moving the proximal and distal piezoelectric elements 394, 396 with respect to each other instead.

The proximal piezoelectric element is adapted to also deform in a radial direction with respect to the medical instrument 102 to frictionally engage and release the medical instrument 102.

The pairs of proximal, intermediate, and distal piezoelectric elements 394, 395, 396 are adapted to be independently controlled, e.g. via a control unit of the manipulation module, such that the medical instrument 102 may be sequentially advanced by carrying out the steps shown in Figs. 6A - 6H by successively deforming the piezoelectric elements 394, 395, 396.

Alternatively, to the piezoelectric elements 394, 395, 396 in Figs. 6A - 6H, the translational driver 3 of a manipulation module may comprise a first gripper and a second gripper. The first and second gripper may be actuated for translationally radially engaging and releasing the medical instrument. The translational driver 3 may have a longitudinal translation unit for changing the distance between the first and second gripper in a direction parallel to a pre-defined axis. This allows that the previously described inchworm mechanism in Figs. 6A - 6G may also be performed by translational movement instead of piezoelectric deformation.

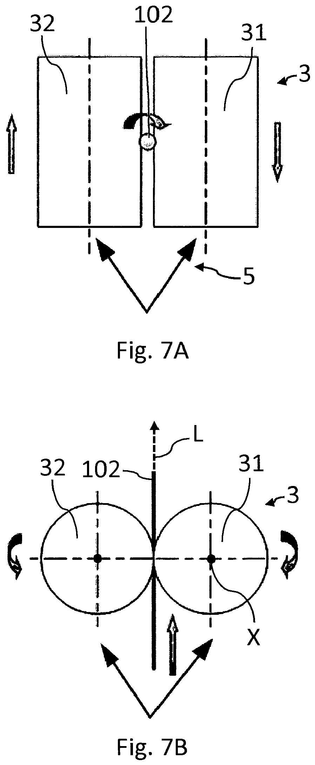

The figures 7A and 7B show a cross-sectional view of an embodiment of the translational driver 3 and a rotational driver 5 of a manipulation module formed by two cylindrical rollers 31, 32 rotating the medical instrument 102 and a longitudinal-sectional view of the cylindrical rollers 31, 32 moving the medical instrument 102 along its longitudinal axis L by rotating the cylindrical rollers 31, 32.

The figure 7A shows that the cylindrical rollers 31, 32 are translationally actuatable independently from each other orthogonally to the longitudinal axis L of the medical instrument 102. The directional arrows in figure 7A show that the cylindrical rollers 31, 32 are moved in opposing parallel directions orthogonally to a longitudinal axis L of the medical instrument 102. The contact surfaces of the cylindrical rollers 31, 32, which contact the medical instrument 102, thus rotate the medical instrument about its longitudinal axis L.

The rotation of the medical instrument via the translational driver 3 in Fig. 7A is controlled by a control unit of the manipulation module (not shown in Figs. 7A or 7B). The control unit may further be adapted to move only one of the cylindrical rollers 31, 32 to adjust the spatial position of the longitudinal axis of the medical instrument 102 by rotating the medical instrument 102 between the contacting surfaces.

The figure 7B shows that the cylindrical rollers 31, 32 of Fig. 7A are further rotatably actuatable in opposing directions around their symmetry axis X for moving the medical instrument 102 along its longitudinal axis L as indicated by the directional arrows. This allows advancing and retracting the medical instrument 102 in a partially or fully automated manner by the cylindrical rollers 31, 32 being controlled via a control unit.

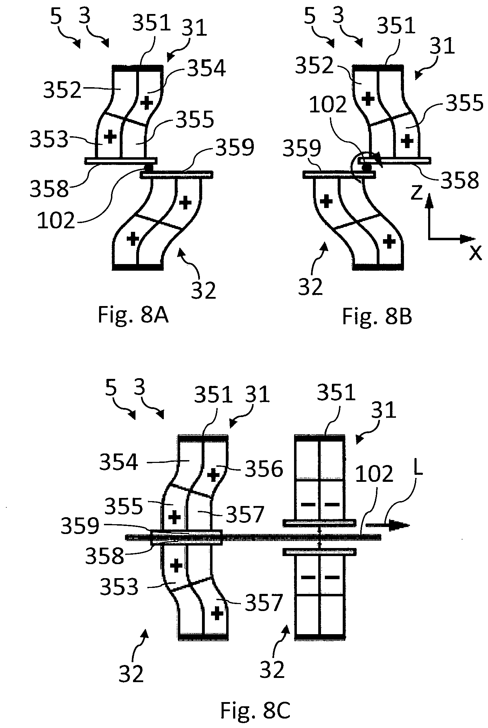

The figures 8A to 8C show a cross-sectional view and a longitudinal-sectional view of an embodiment of the translational driver 3 and the rotational driver 5 of the manipulation module formed by movable contacting surfaces 358, 359 for engaging a medical instrument 102 connected to a solid support 351. The figures 8A to 8C show that the rotational driver 5 and the translational driver 3 are formed by two pairs of driving element 31, 32 respectively which have a plurality of piezoelectric elements 352, 353, 354, 355.

The pairs of driving elements 31, 32 are adapted to engage the medical instrument 102 from two opposing sides. Each driving element 31, 32 has eight piezoelectric elements 352, 353, 354, 355 which are arranged in stacked pairs of four in a rectangular pattern. One pair of four piezoelectric elements 352, 354 is connected to the solid support 351 and the other pair 353, 355 is attached to a contacting surface 358.

The figures 8A and 8B show that the driving elements 31, 32 engage the medical instrument 102 by being bent to an S-shape in opposing directions with respect to the solid support 351. This can be achieved by expanding the diagonally arranged piezoelectric elements 353 and 354 of the first and second driving elements 31, 32 indicated by the plus symbol.

The piezoelectric elements 352, 353, 354, 355 in Figs. 8A and 8B can subsequently be energized in a continuous transition inversely diagonally such that the laterally neighboring piezoelectric elements 352, 355 are energized and expanded instead resulting in an S-shape in the other direction as shown in Fig. 8B. When converting the driving elements 31, 32 the contacting surfaces 358, 359 are moved past each other in opposite directions orthogonally to the medical instrument 102 due to the driving elements 31, 32 being fixed to a solid support 351 respectively. This allows for the medical instrument 102 to be rotated around its longitudinal axis indicated by the bent arrow in Fig. 8B such that the pairs of four piezoelectric elements 352, 353, 354, 355 form the rotational driver 5.

The longitudinal section in figure 8C shows that the two pairs of four piezoelectric elements 354, 355, 356, 357 are adapted to be energized such that the first and second driving elements 31, 32 engage the medical instrument 102 with the contacting surfaces 358, 359 at a proximal location in an S-shape. This can be achieved by the piezoelectric elements 354, 356 of the first driving element 31 being energized to expand in an inverse diagonal manner with respect to the diagonal elements 354, 357 of the second driving element 32 as shown in Fig. 8C. A second pair of driving elements 31, 32 in Fig. 8C radially disengage the medical instrument 102 as indicated by the minus symbols, such that the S-shape of the first and second driving elements can be converted analogously as shown in Figs. 8A and 8B to translationally move the medical instrument 102 in its longitudinal direction L by moving the contacting surfaces 358, 359 with respect to the solid support 351.

The translational movement of the medical instrument 102 in Fig. 8C may be controlled by a control unit which is adapted to energize the two pairs of driving elements 31, 32 of the translational driver 3 such that the medical instrument 102 is translationally moved along its longitudinal axis L similarly to the previously described inchworm mechanism (6A - 6H).

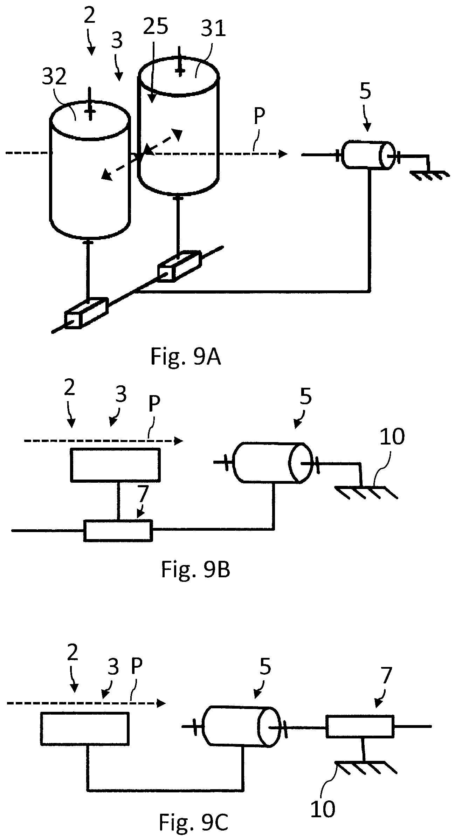

The figure 9A shows a schematic representation of an embodiment of the manipulation unit 2 of the manipulation module having a translational driver 3 and a rotational driver 5 convertible between an engagement configuration (see Figs. 1A and 1B) and a release configuration 25. A first and a second cylindrical element 31, 32 of the translational driver 3 of the manipulation unit 2 are movable by and actuator orthogonally to a pre-defined axis P for converting the manipulation unit 2 between the engagement and release configuration 25, indicated by the dashed double-headed arrows. The cylindrical elements 31, 32 are rotatably drivable for translationally moving a medical instrument along its longitudinal axis in the engagement configuration.

The rotational driver 5 may be a rotational actuator which is adapted for rotating the translational driver 3, optionally together with a centering unit, to rotate the medical instrument (not shown in Fig. 9A). The pre-defined axis P is preferably arranged in a center of rotation of the rotational driver 5. This allows that a medical instrument which is engaged within the manipulation unit 2 in the engagement configuration may be rotated around its longitudinal axis if the longitudinal axis of the medical instrument was aligned with the pre-defined axis P. The driving elements 31, 32 in Fig. 9A may comprise centering surfaces 42 previously described in 3B - 4C.

The figures 9B and 9C show a first and second schematic representation of a manipulation unit 2 of the manipulation module formed by a translational driver 3, a rotational driver 5, and a longitudinal translation unit 7. The translational driver 3 may be motorized such that it is convertible between an engagement and release configuration (see Fig. 9A).

The translational driver 3 may have two driving elements which are adapted to radially engage and release a medical instrument in an engagement configuration and a release configuration 25 (see e.g. Figs. 3A and 3B). The translational driver 3 may not be actively driven for rotating the driving elements as previously described (see Fig. 7B). Instead, the translational driver 3, in particular a motorized gripper, for engaging a medical instrument of different cross-sectional dimensions is coupled to the longitudinal translation unit 7. The longitudinal translation unit 7 in Figs. 9B and 9C is adapted for moving the translational driver 3 in a direction parallel to a pre-defined axis P. This allows that a medical instrument which is radially engaged by the manipulation unit 2 in the engagement configuration may be advanced parallel to the pre-defined axis P.

The figure 9A shows that the longitudinal translation unit 7 may be directly coupled to the translational driver 3 and to the rotational driver 5 which is directly coupled to a solid support 10.

The figure 9B shows that the translational driver 3 may be directly coupled to the rotational driver 5. The rotational driver 5 in Fig. 9B is coupled to the longitudinal translation unit 7 which is directly coupled to the solid support 10.

The translational driver 3 is adapted to be converted between the engagement and release configuration via a control unit of the manipulation module (not shown in Fig. 7). The control unit is further adapted to control the longitudinal translation unit 7 and the rotational driver 5.

## Claims

1. Robotic instrument manipulation module (101) for manipulating medical instruments (102), comprising
a manipulation unit (2) which has at least one translational driver (3) adapted for translationally moving the medical instrument (102) along a longitudinal axis (L) of the medical instrument
and a rotational driver (5) for rotating the medical instrument (102) around its longitudinal axis,
**characterized in that**
the manipulation module (101) has a centering unit (4) which is adapted for aligning the longitudinal axis of the medical instrument (102) in a two-dimensional plane orthogonal to the medical instrument (102) with a pre-defined axis (P), wherein
the manipulation module (101) has a control unit (11) which is adapted for operating the manipulation unit (2), the rotational driver (5), and in particular the centering unit (4) .

2. Manipulation module (101) according to claim 1, wherein the translational driver (3) has a first and a second driving element for engaging the medical instrument (102), in particular formed by a first and a second cylindrical roller (31, 32) which are arrangeable on opposing sides of the medical instrument (102) and are adapted for rotating in opposing directions, in particular around a symmetry axis of the cylindrical rollers (31, 32), for translationally moving the medical instrument (102) along the longitudinal axis (L).

3. Manipulation module (101) according to one of the claims 1 or 2, wherein the rotational driver (5) is adapted for moving a first contacting surface and a second contacting surface of the translational driver (3) in substantially parallel directions substantially orthogonally to the longitudinal axis (L), in particular in opposing parallel directions, for rotating the medical instrument (102).

4. Manipulation module (101) according to one of the preceding claims, wherein the manipulation unit (2) is arranged on a rotatably mounted carrier (6) and the rotational driver (5) is adapted for rotating the carrier (6) and the medical instrument (102) together with the manipulation unit (2), and in particular together with the centering unit (4), in the two-dimensional plane orthogonal to the pre-defined axis (P) .

5. Manipulation module (101) according to one of the preceding claims, wherein the translational driver (3) is formed by at least one of
- a rack pinion system (33), a screw nut system, a snubbing injector (34), an inchworm mechanism (35), or a printer belt (36) for engaging the medical instrument (102) and moving the medical instrument (102) along the longitudinal axis (L),
- a fluid injector and a flow tube (37) which has a fluid inlet (38) for receiving the fluid injector, and
- a rotatable spool (390) or a capstan wheel (391) for holding the medical instrument (102) so that the medical instrument (102) can be translationally moved along the longitudinal axis (L), in particular by the fluid injector.

6. Manipulation module (101) according to one of the preceding claims, wherein the manipulation unit (2) has a longitudinal translation unit (7) such that the translational driver (3) which is adapted for engaging the medical instrument (102), and in particular the centering unit (4), is movable together with the medical instrument (102) in a direction parallel to the pre-defined axis (P).

7. Manipulation module (101) according to one of the preceding claims, wherein the centering unit (4) has a centering structure (41) such that the medical instrument (102) can be translationally moved across the centering structure (41) by the manipulation unit (2) and aligned in the two-dimensional plane orthogonal to the medical instrument (102) with the pre-defined axis (P) extending through the centering structure (41).

8. Manipulation module (101) according to one of the preceding claims, wherein the centering unit (4) is actuatable between a receiving position (47) for receiving the medical instrument (102) and a closed position (46) for substantially aligning the medical instrument with the pre-defined axis (P).

9. Manipulation module (101) according to one of the preceding claims, wherein centering unit (4) has a centering surface (42),
in particular a centering surface (42) which has helical grooves (43), two conical centering surfaces forming a V-shape, or a combination of three rollers arrangeable at different circumferential positions of the medical instrument (102),
which is arranged on the translational driver (3) or formed by the translational driver (3) such that the longitudinal axis of the medical instrument (102) is arranged through a specific location of the centering surface (42) in the two-dimensional plane if the medical instrument (102) is translationally moved along the longitudinal axis (L) by the translational driver (3).

10. Manipulation module (101) according to one of the preceding claims, wherein the manipulation module (101) has one of
- a first and second lateral drivers (8, 9) which are arranged at an angle greater 5° with respect to each other, in particular perpendicular to each other, and are adapted for substantially aligning the longitudinal axis (L) of the medical instrument (102) in the two-dimensional plane orthogonal to the medical instrument (102) with the pre-defined axis (P),
- the rotational driver (5) which is adapted for rotating the medical instrument (102) together with the manipulation unit (2), and one lateral driver (8) which is arranged to move the medical instrument (102), in particular with the manipulation unit (2), in a direction orthogonally to the pre-defined axis, preferably running through a geometric center of the rotational driver (5), such that the rotational driver (5) and the lateral driver (8) are adapted for aligning the longitudinal axis (L) of the medical instrument (102) in the two-dimensional plane with the pre-defined axis (P).

11. Manipulation module (101) according to one of the preceding claims, wherein the manipulation unit (2) is convertible between an engagement configuration (24) for engaging the medical instrument (102) and a release configuration (25) for releasing the medical instrument (102), wherein the control unit (11) and the manipulation unit (2) are adapted for changing the medical instrument (102) by releasing a first medical instrument by converting the manipulation unit (2) from the engagement configuration (24) to the release configuration (25), aligning the manipulation unit (2) with a second medical instrument (102), in particular by relative movement of the manipulation unit (2) with respect to the second medical instrument (103) in a plane orthogonal to the medical instruments (102, 103), and engaging the second medical instrument (103) by converting the manipulation unit (2) from the release configuration (24) to the engagement configuration (25).

12. Manipulation module (101) according to one of the preceding claims, wherein the manipulation module (101) has at least one position sensor for detecting a position of the medical instrument (102) within the two-dimensional plane orthogonal to the medical instrument with respect to the manipulation module (101), in particular with respect to a position within the two-dimensional plane of the translational driver (3).

13. Manipulation module (102) according to one of the claims 11 - 12, wherein the manipulation module (101) has at least one instrument identification sensor for detecting a cross-sectional dimension of the medical instrument engaged by the translational driver (3) in the engagement configuration (24) and the control unit (11) is adapted to determine via the instrument identification sensor a classification of the medical instrument (102) or a longitudinal subsection of a medical instrument (102) with different cross-sectional dimensions at different longitudinal subsections.

14. Manipulation module (101) according to one of the preceding claims, wherein the manipulation module (101) comprises a straightening means for straightening the medical instrument (102), in particular by the control unit (11) being adapted to control an advancement of the medical instrument (102) via the manipulation unit (2) through the centering unit (4) and subsequently retracting the medical instrument (102) through the centering unit (4).

15. Manipulation module (101) according to one of the preceding claims, wherein
the manipulation module (101) has a second manipulation unit (20) which has at least one additional translational driver (30) adapted for translationally moving a second medical instrument (103) along a longitudinal axis of the second medical instrument (103),
and optionally a second rotational driver (50) for rotating the second medical instrument (103) around its second longitudinal axis (L2),
**characterized in that**
the manipulation module (101) has a second centering unit (40) which is adapted for aligning the second longitudinal axis (L2) of the second medical instrument (103) in a two-dimensional plane orthogonal to the second medical instrument (103) with the pre-defined axis (P), wherein
the manipulation module (101) has a control unit (11) which is adapted for operating the first and second manipulation unit (20), the first and preferably the second rotational driver (50), and in particular the first and second centering unit (40).
